# EUROPEAN PATENT APPLICATION

(11) **EP 4 725 435 A1**
(43) Date of publication of application: **15.04.2026**
(21) Application number: 24855754.8
(22) Date of filing: 18.08.2024
(51) Int. Cl.: A61B 18/18, A61N 5/06

(54) **SKIN CARE DEVICE**

(30) Priority: 18.08.2023 CN 202322244701 U
(71) Applicant: Shenzhen Ulike Smart Electronics Co., Ltd., Shenzhen, Guangdong 518000 (CN)
(72) Inventor: HE, Dengshi, Shenzhen, Guangdong 518000 (CN)
(74) Representative: Harris, Oliver John Richard
(86) International application number: PCT/CN2024/112938
(87) International publication number: WO 2025/040031

(57) **Abstract**

A skin care device, comprising a housing (100), a light source assembly (200), and a circuit board assembly (300). The housing (100) is provided with a light outlet (11). The light source assembly (200) comprises a light reflecting member (21) and at least two light sources (22). The circuit board assembly (300) includes a circuit board (31), a first conductive support (32), and a second conductive support (33). The first conductive support (32) is mounted on the circuit board (31) and is connected to one end of each light source (22), and the second conductive bracket (33) is mounted on the circuit board (31) and is connected to another end of each of the light sources (22). The device electrically connects positive electrodes of all of the light sources (22) to the circuit board (31) by means of a same conductive support, and electrically connects negative electrodes to the circuit board (31) by means of a same conductive support, so as to improve the consistency of light emission of all of the light sources (22).

## Description

This application claims the priority of Chinese Patent Application No. 202322244701.7, entitled "skin care device", filed with the China National Intellectual Property Administration on August 18, 2023, the entire content of which is incorporated herein by reference.

### TECHNICAL FIELD

The present application relates to the field of beauty devices, and in particular, to a skin care device.

### BACKGROUND

Hair removal devices and photon rejuvenation devices are commonly used skin care devices in daily life. These skin care devices achieve effects such as hair removal and photon skin rejuvenation on skin of a user, mainly by irradiating it with intense pulsed light (IPL).

### SUMMARY

### Technical problem

In the related technologies, taking a hair removal device as an example, a skin care device generally includes only one lamp tube as an IPL light source, resulting in poor skin care effects produced by the skin care device.

### Technical solution

Embodiments of the present application provide a skin care device, including:
a housing, where a light outlet is formed on the housing;
a light source assembly arranged inside the housing, where the light source assembly includes a light reflector and at least two light sources, the light reflector is at least partially located on a side of the at least two light sources facing away from the light outlet to reflect part of light emitted from the at least two light sources towards the light outlet; and
a circuit board assembly arranged inside the housing, where the circuit board assembly includes a circuit board, a first conductive bracket, and a second conductive bracket, the first conductive bracket is mounted on the circuit board and connected to one end of each light source, the second conductive bracket is mounted on the circuit board and connected to another end of each light source, so that each light source is supported on the circuit board and electrically connected to the circuit board.

### Beneficial Effects

In the embodiments of the present application, the light source assembly includes multiple light sources, so that the light source assembly operates at a high maximum power for skin care, thereby improving the skin care effects produced by the skin care device. Furthermore, positive electrodes of all the light sources are electrically connected to the circuit board through a same conductive bracket and negative electrodes of all the light sources are electrically connected to the circuit board through a same conductive bracket, so that the consistency of current transmission between the circuit board and all the light sources is improved, which improves the consistency of light emission from all the light sources, ultimately improving the skin care effects produced by the skin care device.

### BRIEF DESCRIPTION OF THE DRAWINGS

FIG. 1 is a perspective view of a skin care device according to an embodiment of the present application.
FIG. 2 is a schematic structural view of a circuit board assembly and a light source assembly of the skin care device shown in FIG. 1.
FIG. 3 is another schematic structural view of a circuit board assembly and a light source assembly shown in FIG. 2.
FIG. 4 is a second schematic structural view of a first conductive bracket of the light source assembly shown in FIG. 2.
FIG. 5 is a third schematic structural view of a first conductive bracket of the light source assembly shown in FIG. 2.
FIG. 6 is a second schematic structural view of a second conductive bracket of the light source assembly shown in FIG. 2.
FIG. 7 is a schematic diagram of an arrangement of the light sources in the skin care device shown in FIG. 1.
FIG. 8 is a schematic diagram of another arrangement of the light sources in the skin care device shown in FIG. 1.
FIG. 9 is a first schematic structural view of a light reflector of the skin care device shown in FIG. 1.
FIG. 10 is a second schematic structural view of a light reflector of the skin care device shown in FIG. 1.
FIG. 11 is a schematic diagram showing a positional relationship between the light sources and the light reflector along a length direction in the skin care device shown in FIG. 1.
FIG. 12 is a schematic diagram showing another positional relationship between the light sources and the light reflector along a length direction in the skin care device shown in FIG. 1.
FIG. 13 is a schematic structural diagram of a light source assembly and a circuit board assembly shown in FIG. 2 after being installed with a spacer.
FIG. 14 is a top view of the skin care device shown in FIG. 1.
FIG. 15 is a cross-sectional view of the skin care device shown in FIG. 14 taken along line A-A.
FIG. 16 is an enlarged view of part X in FIG. 15.
FIG. 17 is a cross-sectional view of the skin care device shown in FIG. 14 taken along line B-B.

### DETAILED DESCRIPTION

Hair removal devices and photon rejuvenation devices are commonly used skin care devices in daily life. These skin care devices achieve effects such as hair removal and photon skin rejuvenation on skin of a user, mainly by irradiating it with intense pulsed light (IPL).

In the related technologies, taking a hair removal device as an example, a skin care device generally includes only one lamp tube as an IPL light source, resulting in poor skin care effects produced by the skin care device.

In view of the above, in the embodiments of the present application, a skin care device is provided to improve the skin care effects on the skin. The skin care device is a hair removal device or a rejuvenation device for performing hair removal or skin rejuvenation, respectively. Alternatively, the skin care device is another type of beauty device having hair removal or skin rejuvenation functions, and the embodiments of the present application are not limited thereto.

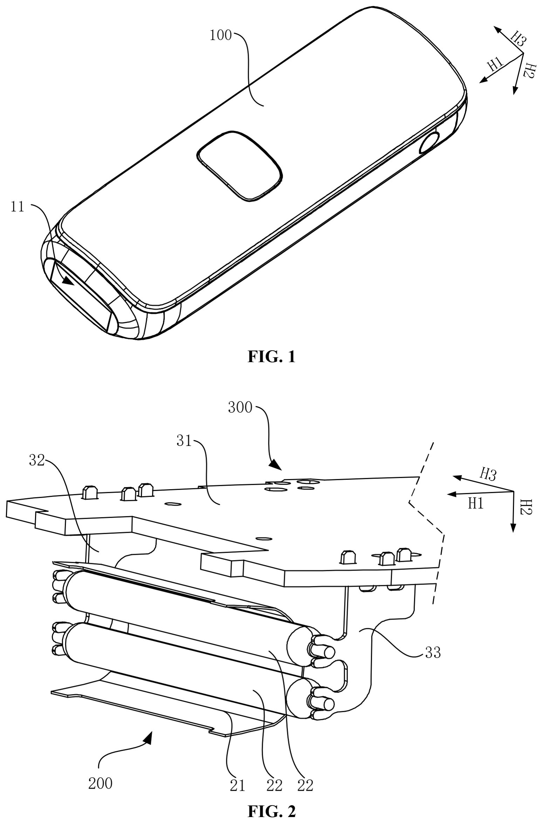

Referring to FIG. 1 and FIG. 2, FIG. 1 is a perspective view of a skin care device according to an embodiment of the present application, and FIG. 2 is a schematic structural view of a circuit board assembly and a light source assembly of the skin care device shown in FIG. 1. The skin care device includes a housing 100, a light source assembly 200, and a circuit board assembly 300.

A light outlet 11 is formed on the housing 100. The light source assembly 200 is arranged inside the housing 100. The light source assembly 200 includes a light reflector 21 and at least two light sources 22. The light reflector 21 is at least partially located on a side of the at least two light sources 22 facing away from the light outlet 11 to reflect part of light emitted from the at least two light sources 22 towards the light outlet 11. Therefore, since the light source assembly 200 according to the embodiment of the present application includes multiple light sources 22, enabling the light source assembly 200 to operate at a high maximum power for skin care, thereby improving the care effects.

The circuit board assembly 300 is arranged inside the housing 100. The circuit board assembly 300 includes a circuit board 31, a first conductive bracket 32, and a second conductive bracket 33. The first conductive bracket 32 is mounted on the circuit board 31 and connected to one end of each light source 22, and the second conductive bracket 33 is mounted on the circuit board 31 and connected to the other end of each light source 22, so that each light source 22 is supported on the circuit board 31 and electrically connected to the circuit board 31.

The end of the light source 22 connected to the first conductive bracket 32 is a positive electrode, and the end of the light source 22 connected to the second conductive bracket 33 is a negative electrode. Alternatively, the end of the light source 22 connected to the first conductive bracket 32 is a negative electrode, and the end of the light source 22 connected to the second conductive bracket 33 is a positive electrode. The embodiments of the present application are not limited thereto.

Therefore, by electrically connecting the positive electrodes of all the light sources 22 to the circuit board 31 through a same conductive bracket and electrically connecting the negative electrodes of all the light sources 22 to the circuit board 31 through a same conductive bracket, the consistency of current transmission between the circuit board 31 and all the light sources 22 is improved. This improves light consistency of all the light sources 22, ultimately improving the care effects of the skin care device.

For example, since the consistency of the light emission across all the light sources 22 is improved, the uniformity of brightness across various positions of the light outlet 11 is improved, thereby avoiding situations where insufficient hair removal or inadequate skin rejuvenation occurs due to low brightness at certain areas of the light outlet 11.

Hereinafter, the technical solution of the embodiments of the present application is explained and described below with reference to the first conductive bracket 32 and the second conductive bracket 33, respectively.

The first conductive bracket 32 is made of a conductive metal material. For example, the first conductive bracket 32 is copper, aluminum, silver, gold, nickel, etc. Alternatively, the first conductive bracket 32 is made of other non-metallic conductive materials, such as graphite or conductive rubber. The embodiments of the present application are not limited thereto.

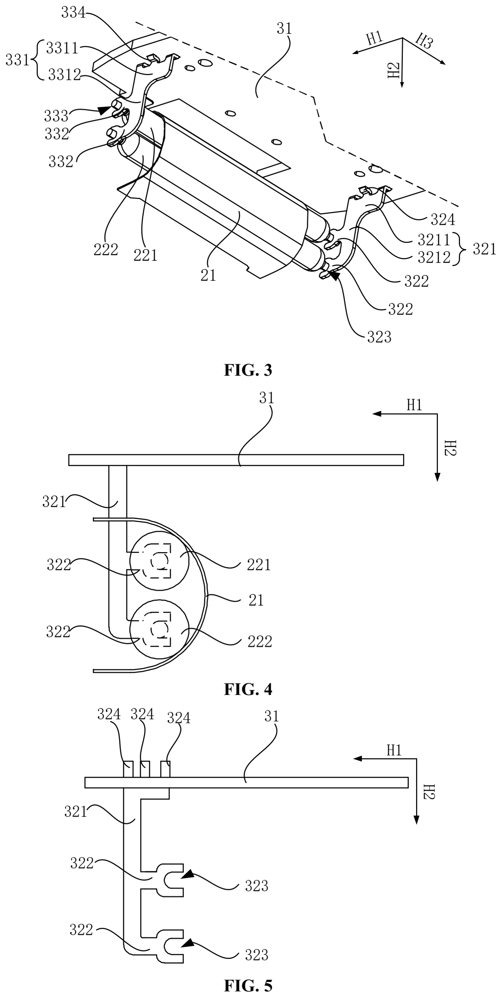

Please continue to refer to FIG. 3 to FIG. 5, FIG. 3 is a second schematic structural view of a first conductive bracket of a light source assembly shown in FIG. 2, FIG. 4 is a second schematic structural view of a first conductive bracket of the light source assembly shown in FIG. 2, and FIG. 5 is a third schematic structural view of a first conductive bracket of the light source assembly shown in FIG. 2. The first conductive bracket 32 includes a first main body portion 321 and multiple first connection portions 322. The first main body portion 321 is mounted on the circuit board 31. The first main body portion 321 is extended from the circuit board 31 toward the at least two light sources 22. Each first connection portion 322 is arranged corresponding to one light source 22. One end of each first connection portion 322 is connected to the first main body portion 321, and the other end is connected to one end of the corresponding light source 22, thereby enabling one end of each light source 22 to be electrically connected to the circuit board 31 through the first conductive bracket 32.

The first main body portion 321 is straight strip-shaped, arc-shaped, bent-shaped, or of other irregular shapes. The embodiments of the present application are not limited thereto.

In some embodiments, the first main body portion 321 includes a first mounting portion 3211 and a first extension portion 3212. The first mounting portion 3211 is fixedly connected to the circuit board 31. The first extension portion 3212 is connected to an end of the first mounting portion 3211 close to the light outlet 11 and is extended from a side of the first mounting portion 3211 facing away from the circuit board 31 toward at least two light sources 22. The first extension portion 3212 is connected to each first connection portion 322.

Therefore, it can also be understood that a part of the first main body portion 321 connected to the circuit board 31 is made wide or large to increase a connection area between the first conductive bracket 32 and the circuit board 31, thereby enhancing connection stability of the first conductive bracket 32 and thus providing more stable support for the light sources 22.

Under a condition that positions of the light sources 22 remain unchanged, a distance between the first extension portion 3212 and the light outlet 11 is also fixed. In this case, compared to connecting the first extension portion 3212 to an end of the first mounting portion 3211 far from the light outlet 11, in the embodiment of the present application, the first extension portion 3212 is connected to a side of the first mounting portion 3211 close to the light outlet 11, allowing the first mounting portion 3211 to be farther from the light outlet 11, thereby avoiding a risk of electric leakage caused by the first mounting portion 3211 being too close to the light outlet 11.

The first mounting portion 3211 includes a free side facing away from the circuit board 31. In the present application, "the first extension portion 3212 is connected to an end of the first mounting portion 3211 close to the light outlet 11" is interpreted as that the first extension portion 3212 is connected to the free side of the first mounting portion 3211 and is offset on that free side in a direction toward the light outlet 11.

It can also be understood that the end of the first mounting portion 3211 close to the light outlet 11 refers to a portion from a middle of the first mounting portion 3211 to an end surface of the first mounting portion 3211 facing the light outlet 11. In the embodiments of the present application, "the first extension portion 3212 is connected to an end of the first mounting portion 3211 close to the light outlet 11" is interpreted as that the first extension portion 3212 is connected to any position between the middle of the first mounting portion 3211 and the end surface of the first mounting portion 3211 facing the light outlet 11.

The meaning of "the first extension portion 3212 is connected to an end of the first mounting portion 3211 close to the light outlet 11" is that: the first extension portion 3212 is connected to the free side of the first mounting portion 3211 and this free side is offset toward a direction close to the light outlet 11.

In some embodiments, multiple first fixing feet 324 are formed at an end of the first conductive bracket 32 close to the circuit board 31. The multiple first fixing feet 324 are all soldered and fixed to the circuit board 31, thereby improving the stability and reliability of the connection between the first conductive bracket 32 and the circuit board 31 through the multiple first fixing feet 324.

For example, the number of the first fixing feet 324 is two, three, four, or five.

It can also be understood that any one first fixing leg 324 is soldered and fixed to the circuit board 31 so as to form only a physical connection, or any one first fixing leg 324 is soldered and fixed to the circuit board 31 and form an electrical connection with the circuit board 31 so as to transmit current. The embodiments of the present application are not limited thereto.

For example, taking the number of the first fixing feet 324 as three as an example, all three first fixing feet 324 are soldered and fixed to the circuit board 31 to form electrical connections with the circuit board 31.

It can also be understood that the multiple first fixing feet 324 are all arranged on the aforementioned first mounting portion 3211. For example, the multiple first fixing feet 324 are arranged on the first mounting portion 3211 along a direction away from the light outlet 11.

In some embodiments, a thickness of the first main body portion 321 is uniform. As a result, an electrical resistance of the first main body portion 321 is uniform. This makes the consistency of the current transmission between the circuit board 31 and each light source 22 be improved, thereby improving the uniformity of brightness across various positions of the light outlet 11.

Alternatively, in some other embodiments, the thickness of the first main body portion 321 varies. The embodiments of the present application are not limited thereto.

In some embodiments, a width of the first main body portion 321 is uniform. As a result, an electrical resistance of the first main body portion 321 is uniform. This makes the consistency of the current transmission between the circuit board 31 and each light source 22 be improved, thereby improving the uniformity of brightness across various positions of the light outlet 11.

Alternatively, in some other embodiments, the width of the first main body portion 321 varies. The embodiments of the present application are not limited thereto.

Only the width of the first main body portion 321is uniform; or only the thickness of the first main body portion 321 is uniform. The embodiments of the present application are not limited thereto. Alternatively, both the width and the thickness of the first main body portion 321 are uniform. As a result, the consistency of the electrical resistance of each first main body portion 321 is improved.

In some embodiments, all the first connection portions 322 are located on a side of the first main body portion 321 close to the light outlet 11, or all the first connection portions 322 are located on a side of the first main body portion 321 facing away from the light outlet 11. The embodiments of the present application are not limited thereto.

In some embodiments, the lengths of all the first connection portions 322 are equal, so that the consistency of electrical resistances of all the first connection portions 322 is improved. This makes the consistency of the current transmission between the circuit board 31 and each light source 22 be improved, thereby improving the uniformity of brightness across various positions of the light outlet 11.

Alternatively, in some other embodiments, the lengths of only some of the first connection portions 322 are equal, or the lengths of all the first connection portions 322 are different. The embodiments of the present application are not limited thereto.

In some embodiments, all the first connection portions 322 have the same thickness, so that all the first connection portions 322 have the same electrical resistance. This makes the consistency of the current transmission between the circuit board 31 and each light source 22 be improved, thereby improving the uniformity of brightness across various positions of the light outlet 11.

Alternatively, in some other embodiments, only some of the first connection portions 322 have the same thickness, or all the first connection portions 322 have different thicknesses. The embodiments of the present application are not limited thereto.

In some embodiments, all the first connection portions 322 have the same width, so that all the first connection portions 322 have the same electrical resistance. This makes the consistency of the current transmission between the circuit board 31 and each light source 22 be improved, thereby improving the uniformity of brightness across various positions of the light outlet 11.

Alternatively, in some other embodiments, only some of the first connection portions 322 have the same width, or all the first connection portions 322 have different thicknesses. The embodiments of the present application are not limited thereto.

All the first connection portions 322 only have the same width; or all the first connection portions 322 only have the same thickness; or the lengths of all the first connection portions 322 are equal; or only the thickness and widths of all the first connection portions 322 are equal; or the thicknesses and lengths of all the first connection portions 322 are equal; or the thicknesses and widths of all the first connection portions 322are equal. The embodiments of the present application are not limited thereto. Alternatively, the lengths, thicknesses, and widths of all the first connection portions 322 are equal respectively. In other words, all the first connection portions 322 have the same shape and size. As a result, the consistency of the electrical resistance of each first main body portion 321 is improved.

It can also be understood that a width and a thickness of the first main body portion 321 are uniform, and all the first connection portions 322 are identical to each other in length, width, and thickness, thereby making the consistency of the current transmission between the circuit board 31 and all the light sources 22 be improved.

Alternatively, it is also possible that the width of the first main body portion 321 and/or the thickness of the first main body portion 321 is uniform, and all the first connection portions 322 are identical to each other in one or more of length, width, and thickness; or, both the width and thickness of the first main body portion 321 vary, and all the first connection portions 322 are identical to each other in one or more of length, width, and thickness; or, both the width and thickness of the first main body portion 321 vary, and all the first connection portions 322 are different with each other in all of length, width, and thickness. The embodiments of the present application are not limited thereto.

In some embodiments, at least two first clamping slots 323 are formed in the first conductive bracket 32, and one end of each light source 22 is clamped into one first clamping slot 323.

After one end of the light source 22 is clamped into the corresponding first clamping slot 323, the light source 22 and the first conductive bracket 32 are fixed by soldering for reinforcement or are not soldered to facilitate replacement. The embodiments of the present application are not limited thereto.

Continuing in conjunction with the aforementioned structure where the first conductive bracket 32 includes multiple first connection portions 322, one first clamping slot 323 is formed in an end of each first connection portion 322 away from the first main body portion 321.

In some embodiments, the second conductive bracket 33 is made of a conductive metal material. For example, the second conductive bracket 33 is copper, aluminum, silver, gold, nickel, etc. Alternatively, the second conductive bracket 33 is made of other non-metallic conductive materials, such as graphite or conductive rubber. The embodiments of the present application are not limited thereto.

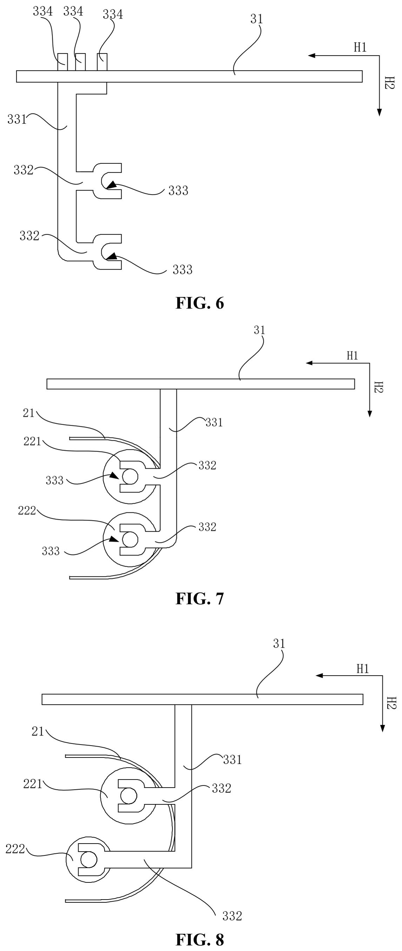

Please continue to refer to FIG. 6, FIG. 6 is a second schematic structural view of a second conductive bracket of the light source assembly shown in FIG. 2. The second conductive bracket 33 includes a second main body portion 331 and multiple second connection portions 332. The second main body portion 331 is mounted on the circuit board 31. The second main body portion 331 is extended from the circuit board 31 toward at least two light sources 22. Each second connection portion 332 is arranged corresponding to one light source 22. One end of each second connection portion 332 is connected to the second main body portion 331, and the other end of each second connection portion 332 is connected to one end of the corresponding light source 22, thereby enabling one end of each light source 22 to be electrically connected to the circuit board 31 through the second conductive bracket 33.

The second main body portion 331 is straight strip-shaped, arc-shaped, bent-shaped, or of other irregular shapes. The embodiments of the present application are not limited thereto.

In some embodiments, the second main body portion 331 includes a second mounting portion 3311 and a second extension portion 3312. The second mounting portion 3311 is fixedly connected to the circuit board 31. The second extension portion 3312 is connected to an end of the second mounting portion 3311 close to the light outlet 11 and is extended from a side of the second mounting portion 3311 facing away from the circuit board 31 toward at least two light sources 22. The second extension portion 3312 is connected to each first connection portion 322.

Therefore, it can also be understood that a part of the second main body portion 331 connected to the circuit board 31 is made wide or large to increase a connection area between the second conductive bracket 33 and the circuit board 31, thereby enhancing a connection stability of the second conductive bracket 33 and thus providing more stable support for the light sources 22.

Under a condition that the positions of the light sources 22 remain unchanged, a distance between the second extension portion 3312 and the light outlet 11 is also fixed. In this case, compared to connecting the second extension portion 3312 to an end of the second mounting portion 3311 far from the light outlet 11, in the embodiment of the present application, the second extension portion 3312 is connected to a side of the second mounting portion 3311 close to the light outlet 11, allowing the second mounting portion 3311 to be farther from the light outlet 11, thereby avoiding a risk of electric leakage caused by the second mounting portion 3311 being too close to the light outlet 11.

The second mounting portion 3311 includes a free side facing away from the circuit board 31. In the embodiments of the present application, "the second extension portion 3312 is connected to an end of the second mounting portion 3311 close to the light outlet 11" is interpreted as that the second extension portion 3312 is connected to the free side of the second mounting portion 3311 and is offset on that free side in a direction toward a direction close to the light outlet 11.

It can also be understood that the end of the second mounting portion 3311 close to the light outlet 11 refers to a portion between a middle of the second mounting portion 3311 and an end surface of the second mounting portion 3311 facing the light outlet 11. In the embodiments of the present application, "the second extension portion 3312 is connected to an end of the second mounting portion 3311 close to the light outlet 11" is interpreted as that the second extension portion 3312 is connected to any position between the middle of the second mounting portion 3311 and the end surface of the second mounting portion 3311 facing the light outlet 11.

Alternatively, in some other embodiments, the second extension portion 3312 is connected to an end of the second mounting portion 3311 away from the light outlet 11. The embodiments of the present application are not limited thereto.

In some embodiments, multiple second fixing feet 334 are formed at an end of the second conductive bracket 33 close to the circuit board 31. The multiple second fixing feet 334 are all soldered and fixed to the circuit board 31, thereby improving the stability and reliability of the connection between the second conductive bracket 33 and the circuit board 31 through the multiple second fixing feet 334.

For example, the number of second fixing feet 334 is two, three, four, or five.

It can also be understood that any one second fixing leg 334 is soldered and fixed to the circuit board 31 so as to form a physical connection only, or any one second fixing leg 334 is soldered and fixed to the circuit board 31 and form an electrical connection with the circuit board 31 so as to transmit current. The embodiments of the present application are not limited thereto.

For example, taking the number of second fixing feet 334 as three as an example, all three second fixing feet 334 are soldered and fixed to the circuit board 31 to form electrical connections with the circuit board 31.

It can also be understood that the multiple second fixing feet 334 are all arranged on the aforementioned second mounting portion. For example, the multiple second fixing feet 334 are arranged on the second mounting portion along a direction away from the light outlet 11.

In some embodiments, a thickness of the second main body portion 331 is uniform. As a result, an electrical resistance of the second main body portion 331 is uniform. This makes the consistency of the current transmission between the circuit board 31 and each light source 22 be improved, thereby improving the uniformity of brightness across various positions of the light outlet 11.

Alternatively, in some other embodiments, the thickness of the second main body portion 331 varies. The embodiments of the present application are not limited thereto.

In some embodiments, a width of the second main body portion 331 is uniform. As a result, an electrical resistance of the second main body portion 331 is uniform. This makes the consistency of the current transmission between the circuit board 31 and each light source 22 be improved, thereby improving the uniformity of brightness across various positions of the light outlet 11.

Alternatively, in some other embodiments, the width of the second main body portion 331 varies. The embodiments of the present application are not limited thereto.

Only the width of the second main body portion 331 is uniform; or only the thickness of the second main body portion 331 is uniform. The embodiments of the present application are not limited thereto. Alternatively, both the width and the thickness of the second main body portion 331 also are uniform. As a result, the consistency of the electrical resistance of each second main body portion 331 is improved.

In some embodiments, all the second connection portions 332 are located on a side of the second main body portion 331 close to the light outlet 11, or all the second connection portions 332 are located on a side of the second main body portion 331 facing away from the light outlet 11. The embodiments of the present application are not limited thereto.

In some embodiments, the lengths of all the second connection portions 332 are equal, so that the electrical resistance at each second connection portion 332 is equal. This makes the consistency of the current transmission between the circuit board 31 and each light source 22 be improved, thereby improving the uniformity of brightness across various positions of the light outlet 11.

Alternatively, in some other embodiments, the lengths of only some of the second connection portions 332 are equal, or the lengths of all the second connection portions 332 are different. The embodiments of the present application are not limited thereto.

In some embodiments, all the second connection portions 332 have the same thickness, so that the electrical resistance at each second connection portion 332 is the same. This makes the consistency of the current transmission between the circuit board 31 and each light source 22 be improved, thereby improving the uniformity of brightness across various positions of the light outlet 11.

Alternatively, in some other embodiments, only some of the second connection portions 332 have the same thickness, or all the second connection portions 332 have different thicknesses. The embodiments of the present application are not limited thereto.

In some embodiments, all the second connection portions 332 have the same width, so that the electrical resistance at each second connection portion 332 is equal. This makes the consistency of the current transmission between the circuit board 31 and each light source 22 be improved, thereby improving the uniformity of brightness across various positions of the light outlet 11.

Alternatively, in some other embodiments, only some of the second connection portions 332 have the same width, or all the second connection portions 332 have different thicknesses. The embodiments of the present application are not limited thereto.

Only the widths of all the second connection portions 332 are equal; or only the thicknesses of all the second connection portions 332 are equal; or only the lengths of all the second connection portions 332 are equal; or only the thicknesses and widths of all the second connection portions 332 are equal; or only the thicknesses and lengths of all the second connection portions 332 are equal; or only the thicknesses and widths of all the second connection portions 332 are equal. The embodiments of the present application are not limited thereto. Alternatively, the lengths, thicknesses, and widths of all the second connection portions 332 are equal respectively, or in other words, all the second connection portions 332 have the same shape and size. As a result, the consistency of the electrical resistance at each second connection portion 332 is improved.

It can also be understood that the width and the thickness of the second main body portion 331 are uniform, and the lengths, widths, and thicknesses of all the second connection portions 332 are equal respectively, thereby making the consistency of the current transmission between the circuit board 31 and all the light sources 22 be improved.

Alternatively, it is also possible that the width of the second main body portion 331 and/or the thickness of the second main body portion 331 is uniform, and all the second connection portions 332 are identical to each other in one or more of length, width, and thickness; or, both the width and thickness of the second main body portion 331 vary, and all the second connection portions 332 are identical to each other in one or more of length, width, and thickness; or, both the width and thickness of the second main body portion 331 vary, and all the second connection portions 332 are different with each other in all of length, width, and thickness. The embodiments of the present application are not limited thereto.

In some embodiments, at least two second clamping slots 333 are formed on the second conductive bracket 33, and one end of each light source 22 is clamped into one second clamping slot 333.

After one end of a light source 22 is clamped into the corresponding second clamping slot 333, the light source 22 and the second conductive bracket 33 are fixed by soldering for reinforcement or are not soldered to facilitate replacement. The embodiments of the present application are not limited thereto.

Continuing in conjunction with the aforementioned structure where the second conductive bracket 33 includes multiple second connection portions 332, one second clamping slot 333 is formed on an end of each second connection portion 332 away from the second main body portion 331.

The following provides illustrative explanations of the first conductive bracket 32 and the second conductive bracket 33 in conjunction with at least two light sources 22 and the light reflector 21.

Please continue to refer to FIG. 7, FIG. 7 is a schematic diagram of an arrangement of the light sources in the skin care device shown in FIG. 1. The multiple light sources 22 are parallel to each other. For example, each light source 22 includes a lamp tube, such as a xenon lamp tube, and a length direction of each lamp tube is parallel to the others.

It can be understood that, compared to a disordered arrangement of the multiple light sources 22, in the embodiment of the present application, the multiple light sources 22 parallel to each other make the overall light emission formed by the multiple light sources 22 uniform. Then, continuing with the example where the skin care device is a hair removal device or a skin rejuvenation device, the uniformity of light brightness across various positions of the light outlet 11 is improved, thereby avoiding situations where hair removal or skin rejuvenation is inadequate in certain skin areas due to weak brightness, thus improving the effects of hair removal or skin rejuvenation.

In some embodiments, distances from the multiple light sources 22 to the light outlet 11 are equal. It can be understood that the intensity of light emitted by light source 22 decreases as a radiation distance increases. In the embodiment of the present application, the distances from the multiple light sources 22 to the light outlet 11 are made equal, so that the uniformity of light brightness across various positions of the light outlet 11 is improved. Then, continuing with the example where the skin care device is a hair removal device or a skin rejuvenation device, situations where hair removal or skin rejuvenation is inadequate in certain skin areas due to weak brightness are avoided, thus improving the effects of hair removal or skin rejuvenation.

It can also be understood that the multiple light sources 22 are parallel to each other while the distance from each light source 22 to the light outlet 11 is different; or the multiple light sources 22 are not parallel to each other while the distance from each light source 22 to the light outlet 11 is equal; or the multiple light sources 22 are parallel to each other and the distance from each light source 22 to the light outlet 11 is equal. The embodiments of the present application are not limited thereto.

Alternatively, please continue to refer to FIG. 8, FIG. 8 is a schematic diagram of another arrangement of the light sources in the skin care device shown in FIG. 1. The distances from different light sources 22 to the light outlet 11 are also different.

For example, the at least two light sources 22 include a first light source 221 and a second light source 222. A distance from the first light source 221 to the light outlet 11 is greater than a distance from the second light source 222 to the light outlet 11. In other words, the first light source 221 is closer to the light outlet 11. Then, a power of the first light source 221 is greater than a power of the second light source 222. Thereby, a difference between the intensity of light radiated from the first light source 221 to the light outlet 11 and the intensity of light radiated from the second light source 222 to the light outlet 11 is made small or even zero, thereby improving the uniformity of brightness across various positions of the light outlet 11.

It can be understood here that the number of light sources 22 is three, four, five, or six, etc. The first light source 221 and the second light source 222 are any two of the multiple light sources 22. The embodiments of the present application are not limited thereto.

In the following, the positional arrangement of the multiple light sources 22 is further illustrated with examples in conjunction with the light reflector 21.

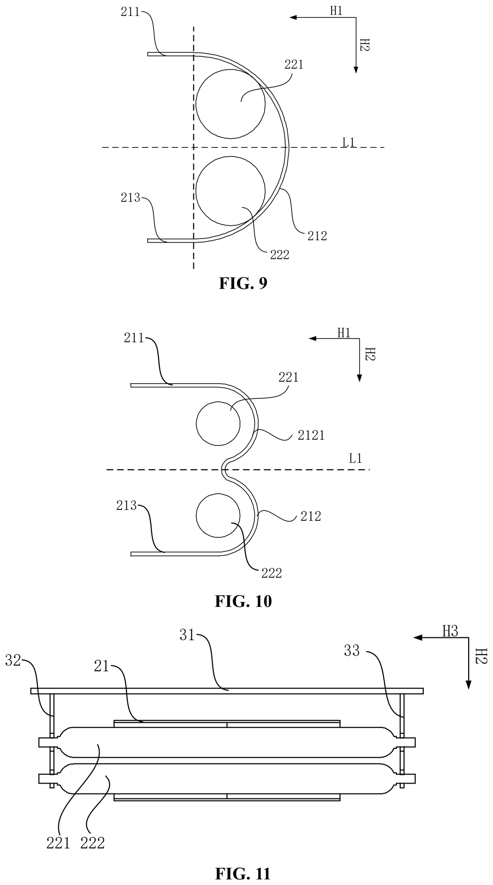

Please continue to refer to FIG. 9, FIG. 9 is a first schematic structural view of a light reflector of the skin care device shown in FIG. 1. A reflective cavity is formed at the light reflector 21. An opening of the reflective cavity faces the light outlet 11. At least two light sources 22 are mounted inside the reflective cavity. In this way, the light emitted by the light sources 22 in 360° directions is reflected and converged toward the light outlet 11 through the reflective cavity to improve the light output efficiency of the light source assembly 200 or increase the brightness at the light outlet 11.

The light outlet 11 is located on a side of the at least two light sources 22 along a first direction H1. The at least two light sources 22 are arranged at intervals along a second direction H2. The second direction H2 is perpendicular to the first direction H1.

It should be noted that a shape of the skin care device is various, for example, it is in a shape of a hair dryer 41, strip, flat small rectangle, circle, or ellipse. For the skin care device in a shape of a (approximately) strip, the first direction H1 is made to correspond to a length direction of the skin care device, and the second direction H2 is made to correspond to a thickness direction of the skin care device. Alternatively, the first direction H1 is made to correspond to the length direction of the skin care device, and the second direction H2 is made to correspond to a width direction of the skin care device. Alternatively, the first direction H1 is made to correspond to the width direction of the skin care device, and the second direction H2 is made to correspond to the thickness direction of the skin care device. The embodiments of the present application are not limited thereto.

In the following, an (approximately) strip skin care device will be primarily used as an example to describe the present application. That is, the embodiments of the present application will continue to use the case where the first direction H1 is the length direction of the skin care device and the second direction H2 is the thickness direction of the skin care device to explain and illustrate the technical solutions of the embodiments of the present application.

In some embodiments, along the second direction H2, the reflective cavity is symmetrical about a first axis L1, where the first axis L1 is parallel to the first direction H1. All the light sources 22 are arranged symmetrically about the first axis L1. Because the reflective cavity is symmetrical about the first axis L1, when the light sources 22 are symmetrically arranged about the first axis L1, the light reflected by an inner wall of the reflective cavity from the multiple light sources 22 is made uniform.

In some embodiments, the light reflector 21 includes a first straight portion 211, a third connection portion 212, and a second straight portion 213 sequentially connected. The first straight portion 211 and the second straight portion 213 are arranged at an interval along the second direction H2, and the third connection portion 212 faces the light outlet 11, so that the first straight portion 211, the third connection portion 212, and the second straight portion 213 are enclosed to form a reflective cavity with an opening facing the light outlet 11. Part of the light emitted by the light sources 22 directly reach the light outlet 11 through the space between the first straight portion 211 and the second straight portion 213, while the other part of the light emitted by the light sources 22 reach the light outlet 11 after being reflected by one or more of the third connection portion 212, the first straight portion 211, and the second straight portion 213.

In some embodiments, the third connection portion 212 includes a curved surface. One end of the curved surface in a circumferential direction is connected to the first straight portion 211, and the other end of the curved surface in the circumferential direction is connected to the second straight portion 213. Alternatively, it can be understood that the first straight portion 211, the second straight portion 213, and the third connection portion 212 form a U-shaped structure, with the third connection portion 212 located at a middle of the U-shape structure. Since the third connection portion 212 is in a shape of a parabola, the light emitted by the multiple light sources 22 is effectively converged, thereby increasing the brightness at the light outlet 11 and improving the uniformity of brightness across various positions of the light outlet 11.

Optionally, please continue to refer to FIG. 10. FIG. 10 is a second schematic structural view of a light reflector of the skin care device shown in FIG. 1. The third connection portion 212 further includes at least two light-converging curved surfaces 2121. Each light-converging curved surface 2121 corresponding to one light source 22. As a result, each light-converging curved surface 2121 provides an excellent light-converging effect for the corresponding light source 22. The embodiments of the present application are not limited thereto.

In some embodiments, the third connection portion 212 is arranged to surround the at least two light sources 22. An end of the third connection portion 212 connected to the first straight portion 211 and an end of the third connection portion 212 connected to the second straight portion 213 are both protruded from a side of the at least two light sources 22 close to the light outlet 11.

Alternatively, it can be simply understood that, along the first direction H1, the multiple light sources 22 are located on an inner side of the third connection portion 212, to allow the third connection portion 212 to provide an effective light-converging effect for the multiple light sources 22 and improve the uniformity of brightness across various positions of the light outlet 11.

It can be understood that the light sources 22 are completely located within a space enclosed by the third connection portion 212. Alternatively, the light sources 22 are partially located within the space enclosed by the third connection portion 212. The embodiments of the present application are not limited thereto.

For example, please continue to refer to FIG. 11. FIG. 11 is a schematic diagram showing a positional relationship between the light sources and the light reflector along a length direction in the skin care device shown in FIG. 1. Continuing with the example where the light source 22 is a lamp tube, the length direction of the lamp tube, the first direction H1, and the second direction H2 are mutually orthogonal. At least one end of the lamp tube along its length direction is located outside the third connection portion 212. Along the length direction of the lamp tube, a length of the portion at each end of the lamp tube located outside the third connection portion 212 is less than or equal to one-fifth of a length of the lamp tube.

For instance, along the length direction of the lamp tube, the length of the portion at each end of the lamp tube located outside the third connection portion 212 is one-fifth, one-sixth, one-seventh, one-eighth of the length of the lamp tube. The embodiments of the present application are not limited thereto.

The length direction of the lamp tube is defined as a third direction H3. Then, continuing with the example of an (approximately) strip skin care device, the length direction of the lamp tube, or the third direction H3, is the width direction of the skin care device.

It can be understood that the brightness at the two ends along the length direction of the lamp tube is usually low during operation. Therefore, placing the lower-brightness parts of the lamp tube outside the light reflector 21 not only avoids excessive waste of the light emitted by the lamp tube but also allows pins of the lamp tube to be located outside the third connection portion 212, facilitating connection with the conductive bracket(s), thereby reducing the assembly and production difficulty of the skin care device.

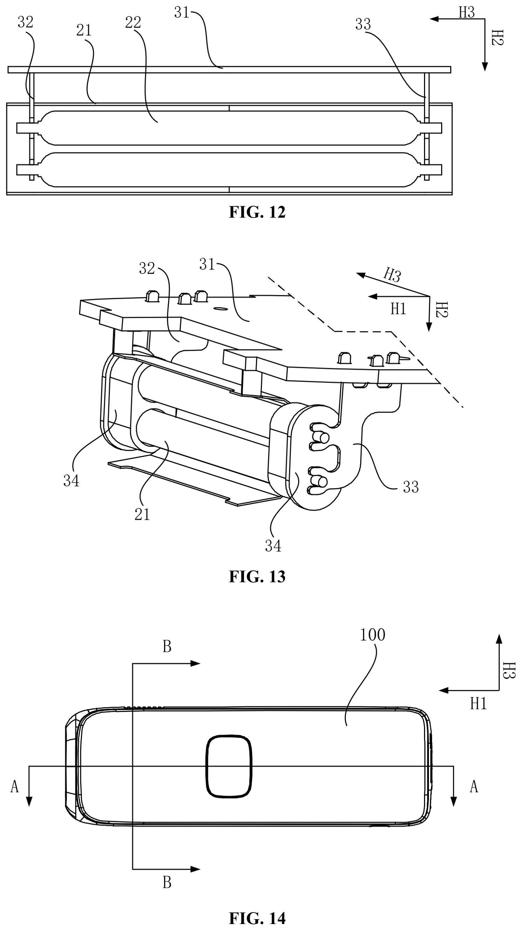

Please continue to refer to FIG. 11 and FIG. 12. FIG. 12 is a schematic diagram showing another positional relationship between the light sources and the light reflector along a length direction in the skin care device shown in FIG. 1. In some embodiments, one end of each light source 22 is located outside the reflective cavity, so that the first conductive bracket 32 is located outside the reflective cavity and connected to one end of each light source 22. Alternatively, one end of each light source 22 is located inside the reflective cavity, so that the first conductive bracket 32 passes through the reflective cavity and is connected to one end of each light source 22.

Likewise, the other end of each light source 22 is located outside the reflective cavity, so that the second conductive bracket 33 is located outside the reflective cavity and connected to the other end of each light source 22. Alternatively, the other end of each light source 22 is located inside the reflective cavity, so that the second conductive bracket 33 passes through the reflective cavity and is connected to the other end of each light source 22. The embodiments of the present application are not limited thereto.

Please continue to refer to FIG. 13. FIG. 13 is a schematic structural diagram of a light source assembly and a circuit board assembly shown in FIG. 2 after being installed with a spacer. Continuing with the example where the light source 22 is a lamp tube, each light source 22 is arranged close to the inner wall of the reflective cavity, and the light reflector 21 is electrically connected to the circuit board 31, so as to allow the circuit board 31 to activate the lamp tube through the light reflector 21. The skin care device further includes a spacer 34. The spacer 34 is sleeved over at least two light sources 22. The spacer 34 separates at least one of the first conductive bracket 32 and the second conductive bracket 33 from the light reflector 21, thereby preventing contact between the light reflector 21 and the conductive bracket(s) which could cause a short circuit or electric leakage.

Alternatively, it is also possible that the light source 22 is a lamp tube, each light source 22 is arranged close to the inner wall of the reflective cavity, and the light reflector 21 is electrically connected to the circuit board 31, so as to allow the circuit board 31 to activate the lamp tube through the light reflector 21. The skin care device further includes a spacer 34. The spacer 34 is sleeved over the first conductive bracket 32, the second conductive bracket 33, or both, and separates the first conductive bracket 32, the second conductive bracket 33, or both, from the light reflector 21, thereby preventing contact between the light reflector 21 and the conductive bracket(s) which could cause a short circuit or electric leakage.

In some embodiments, continuing with the example where the light source 22 is a lamp tube, when at least one end of the lamp tube along its length direction is located outside the third connection portion 212, or in other words, outside the light reflector 21, the spacer 34 is sleeved over the part of the lamp tube located outside the third connection portion 212; or, the spacer 34 is sleeved over the conductive bracket(s).

Below, examples are provided continuing with the scenarios where the conductive bracket is located outside the reflective cavity or penetrates through the light reflector 21.

In some embodiments, at least one end of the light source 22 is located outside the reflective cavity and connected to the corresponding first conductive bracket 32 or second conductive bracket 33. The skin care device further includes a spacer 34. The spacer 34 is sleeved over the light source 22 and located between the light reflector 21 and the first conductive bracket 32 or the second conductive bracket 33, thereby separating the light reflector 21 and the conductive bracket.

In some embodiments, a through-hole in communication with the reflective cavity is formed on the light reflector 21. At least one of the first conductive bracket 32 and the second conductive bracket 33 passes through the through-hole into the reflective cavity to connect to the light source 22. The skin care device further includes a spacer 34. The spacer 34 is at least partially located within the through-hole. The spacer 34 separates an inner wall of the through-hole from the corresponding first conductive bracket 32 and/or second conductive bracket 33, thereby separating the light reflector 21 and the conductive bracket.

For example, continuing with the example where the light source 22 is a lamp tube, when the first conductive bracket 32 and/or the second conductive bracket 33 is penetrated through the light reflector 21 and extended into the reflective cavity to support the light sources 22, the spacer 34 is sleeved over the first conductive bracket 32 and/or the second conductive bracket 33, and the spacer 34 is at least partially located within the through-hole of the light reflector 21.

In this way, the spacer 34 is arranged to separate the light reflector 21 from the first conductive bracket 32 and/or the second conductive bracket 33, to prevent electric leakage from the light reflector 21 to the first conductive bracket 32 and/or the second conductive bracket 33, and to prevent electric leakage from the first conductive bracket 32 and/or the second conductive bracket 33 to the light reflector 21, thereby improving safety during use.

The spacer 23 being sleeved over the at least two light sources 22 includes at least the following two meanings.

It means that the same-side end portions of multiple lamp tubes all penetrate through the same spacer 34. For instance, among the multiple lamp tubes cooperating with the same spacer 34, different lamp tubes penetrate through different isolation holes on the spacer 34 to achieve isolation. Alternatively, different lamp tubes are separated by different spacers 34. The embodiments of the present application are not limited thereto.

It can also be understood that the spacer 34 is made of an insulating material. For example, the spacer 34 is made of rubber. Alternatively, the spacer is made of other insulating materials such as ceramic. The embodiments of the present application are not limited thereto.

In some embodiments, the light sources 22 abut against the light reflector 21, such as the third connection portion 212 of the light reflector 21. Thereby, a gap between the light source 22 and the light reflector 21 is reduced. Consequently, an overall structure of the light source assembly 200 is more compact, facilitating the miniaturization of the skin care device.

Combining with the above example where the light source 22 is a lamp tube, when a space inside the third connection portion 212 is fixed, having the light source 22 directly abut against the third connection portion 212 allows a distance between the first light source 221 and the second light source 222 to be made large, thereby reducing the probability of mutual interference between the first light source 221 and the second light source 222 and improving the reliability of the light sources 22. In other words, it is possible to arrange the first light source 221, the second light source 222, etc., within the light reflector 21 without increasing or only slightly increasing the size of the light reflector 21, thus favoring miniaturized design.

For example, when the at least two light sources 22 include a first light source 221 and a second light source 222, and the third connection portion 212 of the light reflector 21 includes a curved surface, the first light source 221 and the second light source 222 are respectively arranged at two three-equal-part points of the third connection portion 212.

When the number of light sources 22 is greater than three, the multiple light sources 22 are arranged along an inner wall surface of the reflective cavity.

For instance, when the number of the light sources 22 is three and the third connection portion 212 of the light reflector 21 includes a curved surface, the three light sources 22 are respectively located at three four-equal-part points of the third connection portion 212; when the number of the light sources 22 is four and the third connection portion 212 includes a curved surface, the four light sources 22 are respectively located at four five-equal-part points of the third connection portion 212.

The above are some illustrative explanations of the light source assembly 200 and the conductive brackets in the embodiments of the present application. The following continues with illustrative explanations combining some other structures in the embodiments of the present application.

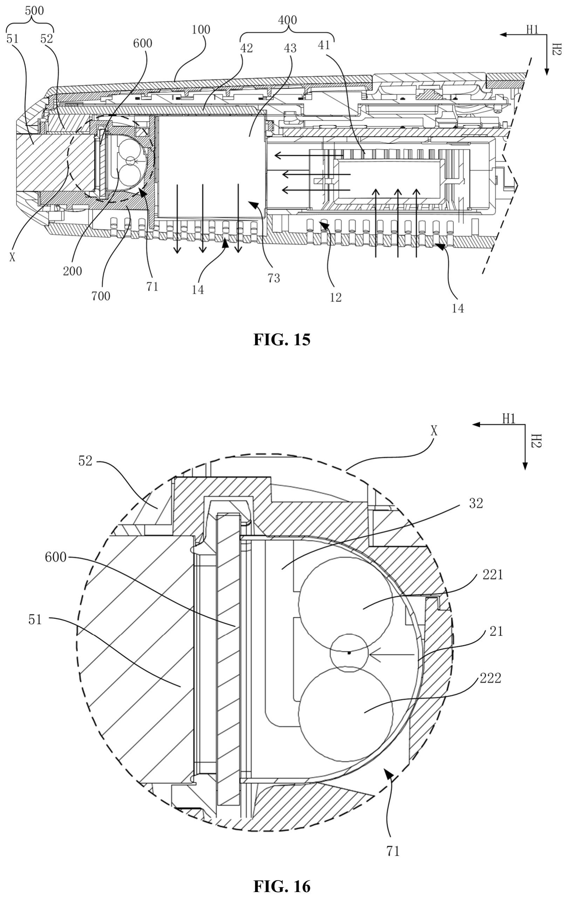

Please continue to refer to FIG. 14 and FIG. 15. FIG. 14 is a top view of the skin care device shown in FIG. 1, and FIG. 15 is a cross-sectional view of the skin care device shown in FIG. 14 taken along line A-A. In some embodiments, the light outlet 11 is located on one side of the at least two light sources 22 along the first direction H1, the circuit board 31 is located on one side of the skin care device along the second direction H2, and the second direction H2 is perpendicular to the first direction H1. The skin care device further includes a heat dissipation assembly 400. The heat dissipation assembly 400 is disposed within the housing 100, and at least part of the heat dissipation assembly 400 is located on a side of the light reflector 21 facing away from the light outlet 11.

It can be understood that since the light sources 22 serve as main heat sources of the skin care device, the heat at the light source assembly 200 increases as the number of light sources 22 increases. Compared to sandwiching the circuit board 31 between the side of the light reflector 21 facing away from the light outlet 11 and the heat dissipation assembly 400, in the embodiments of the present application, the circuit board 31 is arranged on other side of the light sources 22, allowing a distance between the heat dissipation assembly 400 and the light source assembly 200 to be close, thereby shortening the heat dissipation path. This provides a better heat dissipation effect for the light source assembly 200 including the multiple light sources 22, avoiding operational abnormalities caused by excessive heat due to the operation of multiple light sources 22 at the light source assembly 200, and ultimately improving the skin care effect of the skin care device.

The skin care device further includes a cooling assembly 500 mounted on the housing 100 for cooling the skin.

For example, the cooling assembly 500 includes a light guide 51 and a thermoelectric cooling plate 52. The light guide 51 is arranged at the light outlet 11 to transmit light emitted by the light sources 22. The thermoelectric cooling plate 52 is thermally connected to the light guide 51. For example, the thermoelectric cooling plate 52 is in direct contact with the light guide 51 or contacts the light guide 51 via a thermally conductive medium such as silicone-based thermal grease.

In this way, the thermoelectric cooling plate 52 cools the light guide 51, and the cooled light guide 51 is exposed from the light outlet 11 and in contact with the skin to allow the light guide 51 not only to transmit the light from the light sources 22 for skin care for a user but also to be cooled by the thermoelectric cooling plate 52 to provide a cooling effect to the user.

It can also be understood that the light source assembly 200 serves as a main heat source of the skin care device and the light guide 51 is located on a light output side of the light source assembly 200, cooling the light guide 51 through the thermoelectric cooling plate 52 also provides heat-dissipation effect for the light source assembly 200, preventing damage to the light source assembly 200 due to high temperature.

The thermoelectric cooling plate 52, also called a semiconductor thermoelectric cooling plate, utilizes the Peltier effect of semiconductor materials. When a direct current passes through a thermocouple formed by two different semiconductor materials connected in series, heat is absorbed at one end of the thermocouple and released at the other end for the purpose of cooling.

The light guide 51 is made of sapphire or other light-guiding materials. The embodiments of the present application are not limited thereto.

The skin care device further includes an optical filter 600. The optical filter 600 is arranged between the light source assembly 200 and the light guide 51 and is configured to filter light emitted from the light source assembly 200 towards the light guide 51.

For example, the optical filter 600 is configured to transmit light with wavelengths between 420 nm and 1200 nm, thereby forming IPL (intense pulsed light) to irradiate skin of the user. In actual use, the wavelength of light required for hair removal is in a range of 420 nm and 1200 nm, and the wavelength of light required for skin rejuvenation is in a range of 600 nm and 1200 nm. Therefore, the skin care device provides all of cooling, skin rejuvenation, and hair removal functions.

Hereinafter, the technical solutions of the embodiments of the present application are further illustrated with reference to the heat dissipation assembly 400 in the embodiments of the present application.

A heat dissipation channel 12 is arranged inside the housing 100. The heat dissipation assembly 400 includes a fan 41. The fan 41 is configured to drive air in the heat dissipation channel 12 to flow in a preset direction, so as to dissipate heat from the light source assembly 200 and/or the cooling assembly 500.

That is, the fan 41 is configured to dissipate heat only from the light source assembly 200, or to dissipate heat only from the cooling assembly 500, or to dissipate heat from both the light source assembly 200 and the cooling assembly 500. The embodiments of the present application are not limited thereto.

For example, when the heat dissipation assembly 400 is configured to dissipate heat from the cooling assembly 500, the heat dissipation assembly 400 further includes a heat-conducting member 42 and a heat sink 43. The heat-conducting member 42 includes a first end and a second end. The first end is thermally connected to the thermoelectric cooling plate 52. For example, the first end is in direct contact with the thermoelectric cooling plate 52 or contacts with the thermoelectric cooling plate via a thermally conductive medium, such as silicone-based thermal grease. The heat sink 43 is thermally connected to the second end. For example, the first end is in direct contact with the thermoelectric cooling plate 52 or contacts with the heat sink 43 via a thermally conductive medium, such as silicone-based thermal grease. The heat sink 43 is located on a side of the light reflector 21 facing away from the light outlet 11 and at least partially within the heat dissipation channel 12.

In this way, the heat-conducting member 42 transfers heat from the thermoelectric cooling plate 52 to the heat sink 43, and the fan 41 rapidly air-cools the heat sink 43 by driving air in the heat dissipation channel 12 to flow.

Further, when the heat dissipation assembly 400 is configured to dissipate heat from both the light source assembly 200 and the cooling assembly 500, the heat dissipation channel 12 includes a first heat dissipation channel and a second heat dissipation channel.

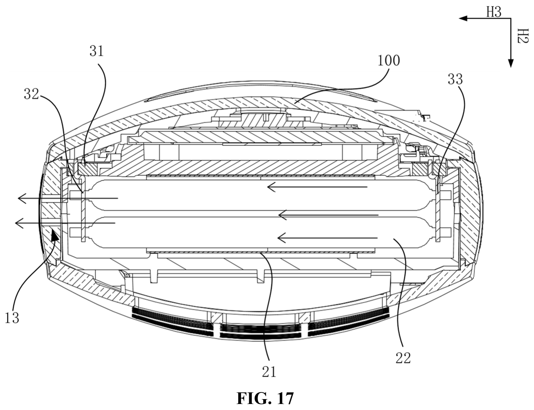

Continuing to refer to FIG. 16 and FIG. 17, FIG. 16 is an enlarged view of part X in FIG. 15. FIG. 17 is a cross-sectional view of the skin care device shown in FIG. 14 taken along line B-B. The first heat dissipation channel is connected between an air port of the housing 100, such as a first air port 13, and the fan 41. The light sources 22 and/or the light reflector 21 is located within the first heat dissipation channel, so that the fan 41 dissipates heat from the light sources 22 and/or the light reflector 21 within the first heat dissipation channel.

The second heat dissipation channel is connected between an air port of the housing 100, such as a second air port 14, and the fan 41. The heat sink 43 is located in the second heat dissipation channel. The heat-conducting member 42 transfers heat from the thermoelectric cooling plate 52 to the heat sink 43, and the second heat dissipation channel is air-cooled by the fan 41 for the purpose of heat dissipation.

In some embodiments, the second heat dissipation channel is independent of the first heat dissipation channel to achieve zoned cooling within the skin care device, thereby ensuring an effective heat dissipation for both the light source assembly 200 and the cooling assembly 500.

Alternatively, the second heat dissipation channel and the first heat dissipation channel are partially interconnected. The embodiments of the present application are not limited thereto.

By way of example, the skin care device further includes a mounting bracket 700. The mounting bracket 700 is arranged within the housing 100. The mounting bracket 700 includes a first mounting cavity 71, a connection channel 72, and a second mounting cavity 73.

The light sources 22 and the light reflector 21 are located within the first mounting cavity 71. One end of the connection channel 72 is in communication with the first mounting cavity 71, and the other end of the connection channel 72 is connected to an air outlet of the fan 41, so that the first mounting cavity 71 and the connection channel 72 form at least a portion of the first heat dissipation channel.

The heat sink 43 is at least partially arranged within the second mounting cavity 73. The second mounting cavity 73 is in communication with the air outlet of the fan 41 to form at least a portion of the second heat dissipation channel.

The mounting bracket 700 includes at least two brackets. For example, the mounting bracket 700 includes a first bracket and a second bracket. The first bracket and the second bracket are spliced together to form the first mounting cavity 71, the connection channel 72, and the second mounting cavity 73 stated above, so as to reduce the manufacturing difficulty of the mounting bracket 700.

Corresponding slots, ribs, or the like are arranged inside the mounting bracket 700 to facilitate the clamping of the light reflector 21, the optical filter 600, etc. The embodiments of the present application are not limited thereto.

The mounting bracket 700 further includes corresponding openings to allow the light guide 51, the thermoelectric cooling plate 52, etc. to pass through the mounting bracket 700. The embodiments of the present application are not limited thereto.

The fan 41 is a centrifugal fan 41, an axial fan 41, or a cross-flow fan 41. The embodiments of the present application are not limited thereto.

For example, when the fan 41 is a centrifugal fan 41, an air inlet of a volute of the centrifugal fan 41 is in communication with an air port of the housing 100, such as a third air port 15. An inlet of the connection channel 72 and an inlet of the second mounting cavity 73 are arranged in parallel at the air outlet of the volute of the centrifugal fan 41, so that the centrifugal fan 41 drives air outside the housing 100 to enter the volute of the centrifugal fan 41. The air is then divided into two paths from the volute of the centrifugal fan 41, that is, one path passes through the connection channel 72 and the first mounting cavity 71 to be discharged for air cooling of the light sources 22 and/or the light reflector 21, and the other path passes through the second mounting cavity 73 for air cooling of the heat sink 43 or the cooling assembly 500.

In some embodiments, the heat sink 43 includes multiple fins. The fins are protruded from a side of the second end along the second direction H2 and are arranged within the heat dissipation channel 12. The fan 41 is located on a side of the fins facing away from the light reflector 21 along the first direction H1. A height of each of the fins along the second direction H2 is greater than or equal to a height of the light source assembly 200 along the second direction H2.

It can be understood that, compared with the arrangement where the fan 41 and the fins are arranged along the second direction H2 behind the light source assembly 200, in the embodiments of the present application, it can be understood that the fan 41 is moved in a direction away from the light source assembly 200, which allows the fins to be made large along the second direction H2, thereby increasing a contact area between the heat sink 43 and the air and enhancing the heat dissipation performance of the heat sink 43.

## Claims

1. A skin care device, comprising:
a housing, a light outlet being formed on the housing;
a light source assembly arranged inside the housing, the light source assembly comprising a light reflector and light sources, and the light reflector being at least partially located on a side of the light sources facing away from the light outlet to reflect part of light emitted from the light sources towards the light outlet; and
a circuit board assembly arranged inside the housing, the circuit board assembly comprising a circuit board, a first conductive bracket, and a second conductive bracket, the first conductive bracket being mounted on the circuit board and connected to one end of each of the light sources, and the second conductive bracket being mounted on the circuit board and connected to another end of each of the light sources, so that each of the light sources is supported on the circuit board and electrically connected to the circuit board.

2. The skin care device according to claim 1, wherein the first conductive bracket comprises:
a first main body portion mounted on the circuit board, the first main body portion being extended from the circuit board toward the light sources; and
a plurality of first connection portions, each of the first connection portions being arranged corresponding to one of the light sources, one end of each of the first connection portions being connected to the first main body portion, and another end of the first connection portion being connected to one end of a corresponding one of the light sources.

3. The skin care device according to claim 2, wherein,
lengths of all the first connection portions are equal; and/or
thicknesses of all the first connection portions are equal; and/or
widths of all the first connection portions are equal; and/or
a thickness of the first main body portion is uniform; and/or
a width of the first main body portion is uniform.

4. The skin care device according to claim 2, wherein a first clamping slot is formed at one end of each of the first connection portions away from the first main body portion, and one end of each of the light sources is clamped into the first clamping slot of a corresponding one of the first connection portions.

5. The skin care device according to claim 2, wherein the first main body portion comprises:
a first mounting portion fixedly connected to the circuit board, and
a first extension portion connected to an end of the first mounting portion close to the light outlet, and extending from a side of the first mounting portion facing away from the circuit board toward the light sources, and the first extension portion being connected to each of the first connection portions.

6. The skin care device according to claim 1, wherein a plurality of first fixing feet are formed at an end of the first conductive bracket close to the circuit board, and the plurality of first fixing feet are all soldered and fixed to the circuit board.

7. The skin care device according to claim 1, wherein,
the light sources are parallel to each other; and/or
distances between the light sources and the light outlet are equal.

8. The skin care device according to claim 1, wherein the light sources comprise a first light source and a second light source, a distance between the first light source and the light outlet is greater than a distance between the second light source and the light outlet, and a power of the first light source is greater than a power of the second light source.

9. The skin care device according to claim 1, wherein a reflective cavity is formed at the light reflector, an opening of the reflective cavity faces the light outlet, and the light sources are mounted inside the reflective cavity;
wherein the light outlet is located on a side of the light sources along a first direction, the light sources are arranged at intervals along a second direction, and the second direction is perpendicular to the first direction.

10. The skin care device according to claim 9, wherein,
along the second direction, the reflective cavity is symmetrical about a first axis, the first axis is parallel to the first direction, and all the light sources are arranged symmetrically about the first axis; and/or
in response to a number of the light sources being greater than three, along the second direction, the light sources are arranged along an inner wall surface of the reflective cavity.

11. The skin care device according to claim 9, wherein,
the light reflector comprises a first straight portion, a third connection portion, and a second straight portion sequentially connected, the first straight portion and the second straight portion are arranged at an interval along the second direction, and the third connection portion faces the light outlet, so that the first straight portion, the third connection portion, and the second straight portion are enclosed to form the reflective cavity.

12. The skin care device according to claim 11, wherein
the third connection portion comprises a curved surface, one end of the curved surface in a circumferential direction is connected to the first straight portion, and another end of the curved surface in the circumferential direction is connected to the second straight portion; or
the third connection portion comprises at least two light-converging curved surfaces, and each of the light-converging curved surfaces is arranged corresponding to one of the light sources.

13. The skin care device according to claim 11, wherein the third connection portion is arranged to surround the light sources, and an end of the third connection portion connected to the first straight portion and an end of the third connection portion connected to the second straight portion are both protruded from a side of the light sources close to the light outlet.

14. The skin care device according to claim 13, wherein the light sources are lamp tubes, any two of a length direction of the lamp tubes, the first direction, and the second direction are mutually orthogonal, and at least one end of the lamp tubes along the length direction is located outside the connection portion;
wherein, along the length direction of the lamp tubes, a length of a portion at each end of each of the lamp tubes located outside the connection portion is less than or equal to one-fifth of a length of each of the lamp tubes.

15. The skin care device according to claim 11, wherein each of the light sources abuts against the connection portion.

16. The skin care device according to claim 15, wherein the light sources are lamp tubes, the light reflector is electrically connected to the circuit board, so as to allow the circuit board to activate the lamp tubes through the light reflector.

17. The skin care device according to claim 9, wherein one end of each of the light sources is located outside the reflective cavity, so that the first conductive bracket is located outside the reflective cavity and connected to one end of each of the light sources; or
one end of each of the light sources is located inside the reflective cavity, so that the first conductive bracket passes through the reflective cavity and is connected to one end of each of the light sources.

18. The skin care device according to claim 9, wherein the light sources are lamp tubes, each of the light sources is arranged close to an inner wall of the reflective cavity, and the light reflector is electrically connected to the circuit board, so as to allow the circuit board to activate the lamp tubes through the light reflector;
wherein the skin care device further comprises a spacer, the spacer is sleeved over light sources, and the spacer is configured to separate the light reflector from the first conductive bracket, the second conductive bracket, or both; or
the skin care device further comprises a spacer, the spacer is sleeved over the first conductive bracket, the second conductive bracket, or both, and the spacer is configured to separate the light reflector from the first conductive bracket, the second conductive bracket, or both.

19. The skin care device according to claim 9, wherein,
at least one end of the light sources is located outside the reflective cavity and connected to a corresponding one of the first conductive bracket and the second conductive bracket; and the skin care device further comprises a spacer sleeved over the light sources and located between the light reflector and the first conductive bracket or the second conductive bracket; or
a through-hole in communication with the reflective cavity is formed on the light reflector, the first conductive bracket, the second conductive bracket, or both passes through the through-hole into the reflective cavity and is connected to the light sources; the skin care device further comprises a spacer, the spacer is at least partially located in the through-hole, and configured to separate an inner wall of the through-hole from the first conductive bracket, the second conductive bracket, or both.

20. The skin care device according to any one of claims 1 to 19, wherein the light outlet is located on a side of the light sources along a first direction, the circuit board is located inside the skin care device on a side thereof along a second direction, and the second direction is perpendicular to the first direction;
wherein the skin care device further comprises a heat dissipation assembly arranged inside the housing, and the heat dissipation assembly is at least partially located on a side of the light reflector facing away from the light outlet.

21. The skin care device according to claim 20, wherein the skin care device further comprises a cooling assembly mounted on the housing to cool a skin.

22. The skin care device according to claim 21, wherein heat dissipation channels are formed in the housing, and the heat dissipation assembly comprises a fan configured to drive air in the heat dissipation channels to flow in a preset direction, so as to dissipate heat from the light source assembly and/or the cooling assembly.

23. The skin care device according to claim 22, wherein the cooling assembly comprises a light guide and a thermoelectric cooling plate, the light guide is arranged at the light outlet to transmit light emitted from the light sources, and the thermoelectric cooling plate is thermally connected to the light guide; wherein the heat dissipation assembly further comprises:
a heat-conducting member, comprising a first end and a second end, wherein the first end is thermally connected to the thermoelectric cooling plate; and
a heat sink thermally connected to the second end, wherein the heat sink is located on the side of the light reflector facing away from the light outlet and at least partially located within the heat dissipation channels.

24. The skin care device according to claim 23, wherein the heat dissipation channels comprise:
a first heat dissipation channel connected between an air outlet of the housing and the fan, wherein at least one of the light sources and the light reflector is located in the first heat dissipation channel; and
a second heat dissipation channel connected between the air outlet of the housing and the fan, wherein the heat sink is located in the second heat dissipation channel.

25. The skin care device according to claim 24, wherein the skin care device further comprises a mounting bracket arranged within the housing, and a first mounting cavity, a connection channel, and a second mounting cavity are formed in the mounting bracket; wherein,
the light sources and the light reflector are arranged in the first mounting cavity, one end of the connection channel is in communication with the first mounting cavity and another end of the connection channel is in communication with an air outlet of the fan, so that the first mounting cavity and the connection channel form at least part of the first heat dissipation channel; and
the heat sink is at least partially arranged in the second mounting cavity, and the second mounting cavity is in communication with the air outlet of the fan to form at least part of the second heat dissipation channel.

26. The skin care device according to claim 23, wherein the heat sink comprises a plurality of fins, the plurality of fins are arranged to protrude from a side of the second end along the second direction and are located within the heat dissipation channels; and
wherein the fan is located on a side of the fins facing away from the light reflector along the first direction, and a height of one of the fins along the second direction is greater than or equal to a height of the light source assembly along the second direction.

27. The skin care device according to any one of claims 1 to 19, wherein the skin care device is a hair removal device or a skin rejuvenation device.
